# EUROPEAN PATENT SPECIFICATION

(11) **EP 1 700 571 B1**
(45) Date of publication and mention of the grant of the patent: **30.07.2008**
(21) Application number: 03773741.8
(22) Date of filing: 25.11.2003
(51) Int. Cl.: A61B 17/64

(54) **EXTERNAL WRIST-FIXING DEVICE**
EXTERNE HANDGELENKSFIXIERVORRICHTUNG
DISPOSITIF DE FIXATION EXTERNE POUR LE POIGNET

(43) Date of publication of application: 13.09.2006
(73) Proprietor: Instituto Tecnologico de Canarias, S.A. (ITC), 38009 Santa Cruz de Tenerife (ES)
(72) Inventor: ARA PINILLA, Javier, 38009 Santa Cruz de Tenerife (ES); DE LA BARREDA LOPEZ, Guillermo, 38009 Santa Cruz de Tenerife (ES); MONOPOLI FORLEO, Donato, 38009 Santa Cruz de Tenerife (ES)
(74) Representative: PROPI, S.L.
(86) International application number: PCT/ES2003/000598
(87) International publication number: WO 2005/053549

(56) References cited:
- WO-A1-00/38584
- ES-A- 8 703 733
- ES-A2- 2 064 278
- ES-T3- 2 096 321
- FR-A1- 2 805 147
- US-A- 5 653 707

## Description

### PURPOSE OF THE INVENTION

The present invention relates to a device which has been especially conceived to create a rigidifying bridge between the patient's arm and hand, in order to immobilise his/her wrist, in the event of fracture or dislocation of said wrist, for the entire recovery period during which the joint must be immobilised.

Nevertheless, the device is equally applicable to a single bone, such as for instance the ulna or the radius, when there is this type of fracture.

The purpose of the invention is to allow for easy positional regulation of the two ends of the fixer in order that, once the latter is implanted on the patient's arm and hand, the pertinent regulations may be conducted for the relative position between these elements at the wrist level to be the most suitable.

### BACKGROUND OF THE INVENTION

There are known fixers with said purpose, which are also placed on the outer side of the arm and are based on the use of two pairs of nails or screws intended to be fixated to two bones or two parts of a bone, emerging on the outer side of the limb, where each pair of screws or nails receives a fixation block from which a bar transversely emerges; this bar is crowned, in turn, by another block which is substantially distant from the first one, such that the two end blocks are interconnected by means of a third bar, which is perpendicular to the preceding ones and may be fixated by means of stud bolts, thus achieving an essentially rigid structure which stabilises the two elements to be joined.

This solution, which is perfectly valid from the theoretical point of view, in practice leads to problems due to the pronounced distance between the nails that are inserted into the bone and the longitudinal bar that connects the two pairs of nails, which reduces the stability of the fixation.

On the other hand, even though this device fulfils its function in a basically satisfactory manner when it is intended to immobilise the two portions of a fractured bone, such as for instance the ulna or the radius, it is very difficult to apply, and requires great professional expertise, when the purpose is to immobilise the hand to the arm, due to a wrist problem, since, whereas in the first case it is only necessary for the two bone sectors to be aligned, in the second case, depending on the various types of injuries, the hand must adopt different inclinations or orientations with respect to the forearm.

Actually, the document US5653707 is known in the background of this invention whit relatively importance. This document relates to an external skeletal fixation system comprising a first external fixation bar having a longitudinal axis an a substantially hexagonal cross section; a second external fixation bar having a longitudinal axis and a substantially hexagonal cross section; and a connector that interconnets said first and second external fixation bars. This connector includes two members having apertures for receiving said first and second bar, and allows a variable angle between the longitudinal axis of said bars.

In opposite of that, the connector of the present invention comprises an articulator with two holes, in which two bearings housed the bars with the capacity to rotate around themselves, and allowing the bar's position to be changed in any direction.

### DESCRIPTION OF THE INVENTION

The external fixator proposed by the invention resolves in a fully satisfactory manner said problems, since it allows for a perfect positional regulation between the two anatomical elements to be joined, after insertion of the nails into them, and not only with respect to closeness/distance, but also with regard to their relative orientation.

More specifically, in order to achieve this, the fixator is structured on the basis of two supports provided with through-holes for respective pairs of the nails used for insertion into the bone; each of these supports is aided by a bearing and a screw-nut set wherein said screw incorporates a head which approaches a spherical annular configuration in order to allow for the passage of a bar which connects said support with a main articulator.

Said through-holes for the nails are laterally open, by means of a narrow slot which, upon closure, causes the support to be fixated to the nails when the screw-nut set is tightened; during this tightening the screw's spherical head substantially fits into the intermediate bearing, against which the bar laterally presses, thus becoming immobilised.

The main articulator is embodied in an extended body with two identical holes having a concave internal surface, which are connected by means of a longitudinal slot that opens towards both of the articulator's faces. Two spherical bearings are placed on said holes; the bars also pass through the bearings, being pressed and immobilised by means of a tightening screw which is transversely mounted on the articulator; the bars' positioning and, consequently, the distance between the supports may be regulated by acting on said tightening screw.

The two spherical bearings have a diametral slot which makes it possible to tighten and/or loosen the bars for their regulation.

As becomes evident, according to the described structuring, the distance between the two end supports may be regulated at will depending on the position of the bars that are fixated to the main articulator, upon tightening thereof, while these bars may form any angle, since they tilt on the main articulator when the latter is in a loose situation and maintain the selected angular position in a perfectly stable manner when it is definitively tightened.

The fixator's special structuring and configuration not only ensure greater mechanical stability, but also make it possible to use plastic materials to obtain certain parts thereof, with the cost reduction this entails.

### DESCRIPTION OF THE DRAWINGS

In order to complement the description that is being made, and in order to contribute to a better understanding of the invention's characteristics, in accordance with an example of preferred embodiment thereof, as integral part of this description a set of drawings is attached, wherein, for illustration purposes, but not limited thereto, the following has been represented.:

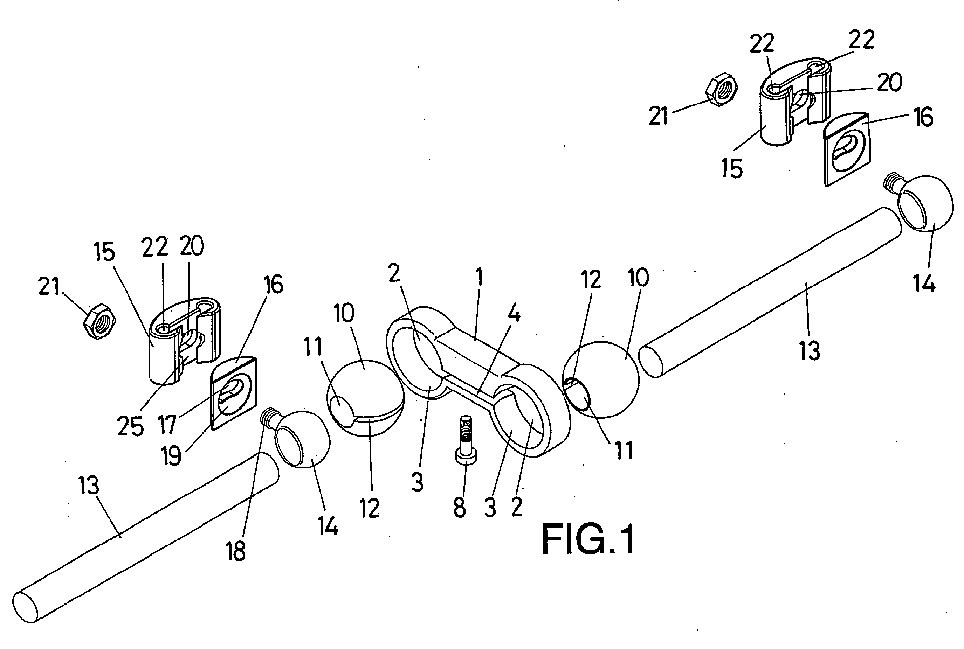

Figure 1.- Shows a perspective breakdown of the parts of an external wrist fixer embodied in accordance with the purpose of this invention.

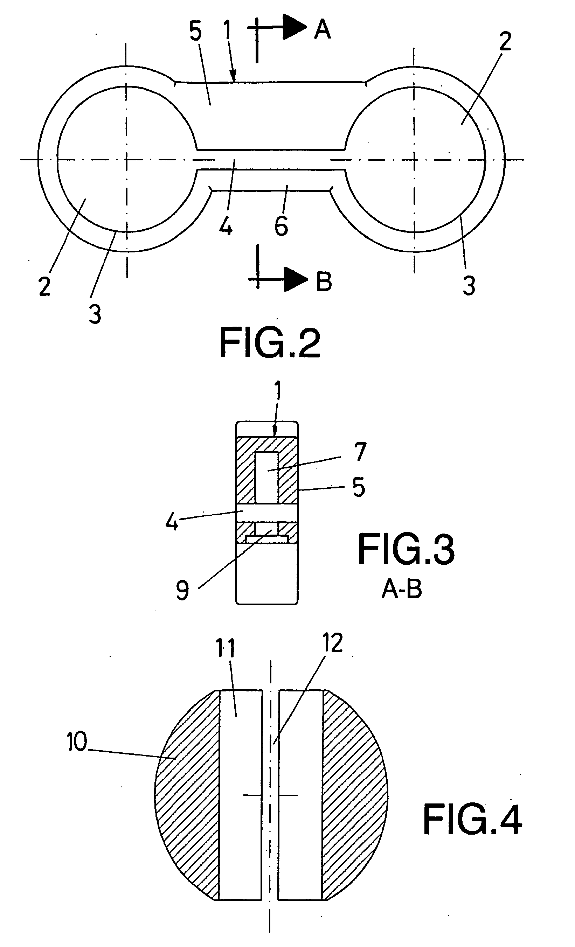

Figure 2.- Shows an elevation view of the main articulator which participates in said device.
Figure 3.- Shows a section detail of the same main articulator.
Figure 4.-Shows a cross-section detail of one of the spherical bearings which also participate in the fixer

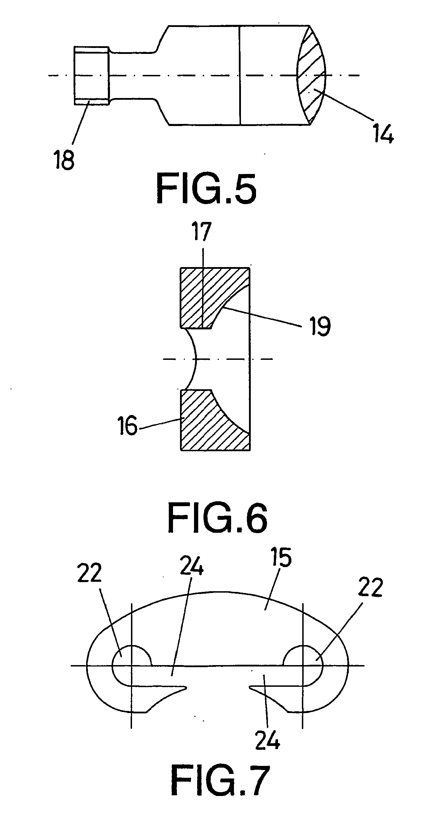

Figure 5.- Shows a section detail of one of the spherical annular head screws.
Figure 6.- Shows a section detail of one of the bearings which intervenes in each of the device's support sets.
Figure 7.- Shows a cross-section detail of one of the fixation supports for the nails.
Figure 8.- Shows, finally, an example of practical use of the fixer, coupled to a patient's forearm and hand, according to a perspective vision.

### PREFERRED EMBODIMENT OF THE INVENTION

In light of said figures, one can observe that the external wrist fixer proposed by the invention is formed by a main articulator (1), embodied in an extended body, which has a pair of identical holes (2) with a concave internal surface (3), creating a cylindrical segment in each case. Both holes (2) are connected by a longitudinal slot (4) that opens towards both of the articulator's (1) sides and is located close to one of the articulator's faces, creating two walls (5) and (6) thereon, the first being thicker and having a threaded, blind hole (7) for a tightening screw (8) that passes through a hole (9) on the thinner wall (6).

Two spherical bearings (10) are placed inside the holes (2) with a concave internal surface (13); the bearings have a diametral through-hole (11) and a slot (12) that diametrically connects each hole (11) to the exterior, such that these spherical bearings (10) may be tightened to a greater or lesser extent by means of the screw (8); this tightening is achieved thanks to the slot (12), since tightening of the screw (8) leads to the articulator's (1) walls (5) and (6) coming closer and, thus, the holes (2) being reduced and, consequently, the spherical bearings (10) being tightened.

One of the bars' (13) ends is housed in the spherical bearings' (10) holes (11); in turn, the bars pass through the main, spherical screws (14), all this in such a way that these bars may be introduced or removed to a greater or lesser extent by simply loosening the tightening screw (8) and performing any of those operations, in order to subsequently fixate it in an undetachable manner by means of the corresponding tightening, as previously mentioned.

In turn, the bars (13) are connected to respective supports (15), which are associated with corresponding bearings (16) provided with a slotted hole (17) in order to allow for tilting, in addition to passage of the threaded shanks (18) pertaining to the spherical screws (14) through them, in addition to the pitch or slotted hole (17), the bearings (16) have a sinkage (19) in the form of a spherical cap, which is intended to partially receive within it the screws' (14) spherical annular head.

The spherical screws' (14) threaded shanks (18), which pass through the bearings' (16) slotted holes (17), in turn pass through another slotted hole (20) provided on each of the supports (15), with tightening being performed by means of the corresponding nut (21).

The supports (15) have the special feature that the parallel holes (22) intended for passage of the respective nails (23) used for insertion into the bone are laterally open by means of respective slots (24), such that their initial diameter is slightly greater than the nails' (23), in order to act as guides upon insertion of the latter into the bone and, following the fixer definitive positioning, tightening of the nuts (21) and, more specifically, leaning of the bearings (16) against the supports' (15) concave-curved front (25) when the slots (24) are closed, pinching of the holes (22) against the nails (23), and, consequently, relative immobilisation between these elements.

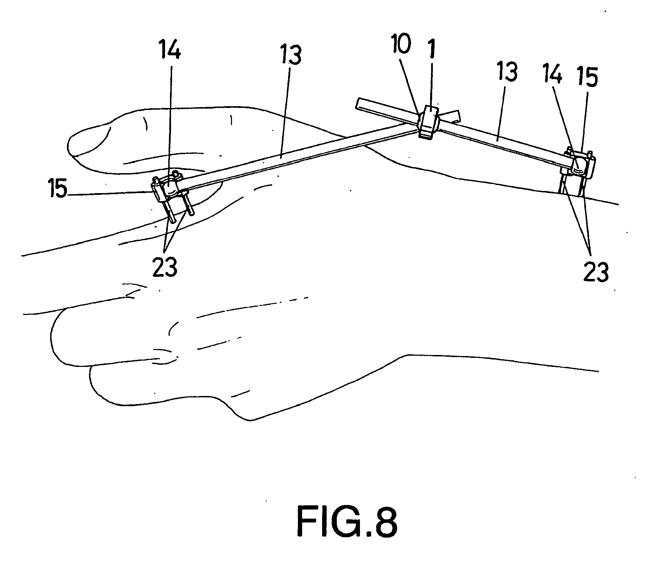

As has been previously mentioned, and as can be deduced from observing figure 8, the fixer makes it possible for the bars (13) to adopt any necessary angularity to be transmitted to the wrist after having inserted the nails (23) into the bone, but it may occur that this angular regulation between bars is not necessary, such as for instance in the case of a bone fracture at the forearm level, in which case it is possible to use a single bar; logically, this makes it unnecessary to use the main articulator (1), in which case the ends of said bar are simply mounted on respective sets of spherical screws (14), bearing (16), and support (15).

## Claims

1. External wrist fixator which, while being especially conceived to act as a bridging element between the forearm and the hand that immobilises the wrist in any selected position, is formed by a main articulator (1), whereto a pair of bars (13) are fixated by one end of both bars (13) which are capable of being regulated both longitudinally and angularly, each of both bars (13) being connected, on the other end, to a support (15) capable of being longitudinally and angularly regulated, and each of said supports (15) being provided with a pair of holes (22) for passage of corresponding nails (23) intended for insertion into the bone; wherein the main articulator (1) is embodied in an extended body provided with a pair of identical first holes (2), **characterised in that** the internal surface of the first holes (2) is concave, creating spherical segments, connected to one another by means of an axial slot (4) which may be narrowed by tightening a transverse screw (8) in order to reduce the corresponding first holes (2), where respective spherical bearings (10) are housed, said bearings including a diametral through-hole (11) that is connected, through a slot (12), with the exterior in order to allow for passage and tightening of the corresponding bar (13), with the latter, in turn, passing through a hole which the head of a spherical screw (14) has for this purpose, and through which it is linked to the respective support (15).

2. External wrist fixator, as claimed in the preceding claim, **characterised in that** the spherical bearings (10) are located on the first holes (2) with the capacity to rotate around themselves, allowing for the bars' (13) position to be changed in any direction.

3. External wrist fixator, as claimed in claim 1, **characterised in that** the supports' (15) holes (22) are connected to the exterior by means of respective lateral slots (24) which, by allowing a slight lateral oversizing of said holes (22) with respect to the nails (23) intended for insertion into the bone, are pinched over said nails when they are definitively fixated to the ends of the corresponding bars (13).

4. External wrist fixator, as claimed in the preceding claims, **characterised in that** the supports' (15) slots (24) open towards a concave curved face (25) on which a bearing (16) traversed by the spherical annular head screw's (14) threaded shank (18) is adapted; these screws (14) are complemented by respective nuts (21)

## Patentansprüche

1. Externe Fixiervorrichtung für Handgelenke, welche, indem diese speziell dazu ausgelegt wurde, als Überbrückungselement zwischen dem Vorderarm und der Hand zu dienen, welches das Handgelenk in jeder ausgewählten Position ruhig stellt, aus einem Hauptartikulator (1) gebildet wird, an dem ein Paar Stangen (13) mit einem Ende beider Stangen (13) befestigt ist, die sowohl der Länge nach als auch bezüglich der Winkel reguliert werden können, wobei jede der beiden Stangen (13) an dem anderen Ende mit einer Stütze (15) verbunden ist, die der Länge nach und bezüglich des Winkels reguliert werden kann, und wobei jede der besagten Stützen (15) mit einem Paar Bohrungen (22) für den Durchgang von entsprechenden Nägeln (23) ausgestattet ist, die für die Einfügung in den Knochen bestimmt sind; bei welcher der Hauptartikulator (1) in einem verlängerten Körper ausgeführt ist, der mit einem Paar identischer erster Bohrungen (2) ausgestattet ist, **dadurch gekennzeichnet, dass** die innere Oberfläche der ersten Bohrungen (2) konkav ist, wobei kugelförmige Segmente gebildet werden, die mittels einer axialen Nut (4) miteinander verbunden sind, die eingeengt werden kann, indem eine quer verlaufende Schraube (8) angezogen wird, um die entsprechenden ersten Bohrungen (2) zu reduzieren, in denen entsprechende Pendellager (10) gelagert sind, wobei die besagten Lager eine diametrale Durchgangsbohrung (11) besitzen, die über eine Nut (12) mit der Außenseite verbunden ist, um einen Durchgang und ein Anziehen der entsprechenden Stange (13) zu ermöglichen, wobei die Letztere wiederum durch eine Bohrung verläuft, die ein Kopf einer Rundschraube (14) zu diesem Zweck besitzt und mittels der diese mit der entsprechenden Stütze (15) verknüpft ist.

2. Externe Fixiervorrichtung für Handgelenke nach dem vorherigen Anspruch, **dadurch gekennzeichnet, dass** die Pendellager (10) in den ersten Bohrungen (2) mit der Fähigkeit gelagert sind, sich um sich selbst zu drehen, wobei es der Position der Stangen (13) ermöglicht wird, in jeder Richtung verändert zu werden.

3. Externe Fixiervorrichtung für Handgelenke nach Anspruch 1, **dadurch gekennzeichnet, dass** die Bohrungen (22) der Stützen (15) mit der Außenseite mittels entsprechender seitlicher Nuten (24) verbunden sind, welche, indem diese eine leichte seitliche Überdimensionierung der besagten Bohrungen (22) gegenüber den Nägel (23) zulassen, die zur Einfügung in den Knochen bestimmt sind, über die besagten Nägel geklemmt werden, wenn diese endgültig mit den Enden der entsprechenden Stangen (13) verbunden werden.

4. Externe Fixiervorrichtung für Handgelenke nach dem vorherigen Anspruch, **dadurch gekennzeichnet, dass** die Nuten (24) der Stützen (15) sich in Richtung einer konkav gebogenen Seite (25) öffnen, auf der ein Lager (16) angepasst ist, das von dem Gewindeschaft (18) des ringförmigen Rundschraubenkopfes (14) durchlaufen wird; diese Schrauben (14) werden durch entsprechende Nuten (21) ergänzt.

## Revendications

1. Fixateur externe de poignet qui, tout en étant spécialement conçu pour servir d'élément en pont entre l'avant-bras et la main qui immobilise le poignet dans n'importe quelle position sélectionnée, est formé d'un articulateur principal (1), et en vue de ceci deux barres (13) sont fixées par une extrémité des deux barres (13) qui peuvent être réglées à la fois longitudinalement et angulairement, chacune des deux barres (13) étant connectée, à l'autre extrémité, à un support (15) pouvant être réglé longitudinalement et angulairement, et chacun desdits supports (15) comportant deux orifices (22) pour le passage des clous correspondants (23) destinés à être insérés dans l'os; dans lequel l'articulateur principal (1) a la forme d'un corps allongé comportant deux premiers orifices identiques (2), **caractérisé en ce que** la surface interne des premiers orifices (2) est concave, créant des segments sphériques, reliés l'un à l'autre au moyen d'une fente axiale (4) qui peut être rétrécie en serrant une vis transversale (8) afin de réduire les premiers orifices correspondants (2), où des coussinets sphériques respectifs (10) sont logés, lesdits coussinets comportant un orifice de passage diamétral (11) qui est relié, à travers une fente (12), avec l'extérieur afin de permettre le passage et la serrage de la barre correspondante (13), avec cette dernière, à son tour, passant dans un orifice que la tête d'une vis sphérique (14) possède à cet effet, et via laquelle elle est reliée au support respectif (15).

2. Fixateur externe de poignet, tel qu'il est revendiqué dans la revendication précédente, **caractérisé en ce que** les coussinets sphériques (10) sont situés sur les premiers orifices (2) avec la capacité de tourner sur eux-mêmes, permettant à la position des barres (13) d'être changée dans n'importe quel sens.

3. Fixateur externe de poignet, tel qu'il est revendiqué dans la revendication 1, **caractérisé en ce que** les orifices (22) des supports (15) sont reliés à l'extérieur au moyen des fentes latérales respectives (24) qui, en permettant un léger surdimensionnement latéral desdits orifices (22) par rapport aux clous (23) destinés à être insérés dans l'os, sont serrées sur lesdits clous lorsqu'ils sont définitivement fixés aux extrémités des barres correspondantes (13).

4. Fixateur externe de poignet, tel qu'il est revendiqué dans les revendications précédentes, **caractérisé en ce que** les fentes (24) des supports (15) s'ouvrent vers une face incurvée concave (25) sur laquelle un coussinet (16) traversé par la tige fileté (18) de vis à tête sphérique annulaire (14) est adapté ; ces vis (14) sont complétés par des écrous respectifs (21).
